# EUROPEAN PATENT APPLICATION

(11) **EP 3 995 159 A1**
(43) Date of publication of application: **11.05.2022**
(21) Application number: 21161909.3
(22) Date of filing: 11.03.2021
(51) Int. Cl.: A61L 15/42, A61L 15/46, A61L 15/58

(54) **COMPOSITE METAL FAR-INFRARED MEDICAL PATCH**

(30) Priority: 05.11.2020 TW 109138695
(71) Applicant: Green Energy Nano Technology Co., Ltd., Taipei City 104 (TW)
(72) Inventor: TIEN-SHOW, LIANG, 104 Taipei City (TW); EN, MENG, 114 Taipei City (TW); SHU-HAN, LIANG, 104 Taipei City (TW); WEN-SHENG, LEE, 104 Taipei City (TW); TSAI, YI-TING, 114 Taipei City (TW); CHEN, HSIANG-CHENG, 114 Taipei City (TW); CHERNG, JUIN-HONG, 105 Taipei City (TW); Shu-Ting, LIANG, 104 TAIPEI CITY (TW); LIN, YI-HSIN, 114 Taipei City (TW)
(74) Representative: dompatent von Kreisler Selting Werner - Partnerschaft von Patent- und Rechtsanwälten mbB

(57) **Abstract**

A patch is provided. The patch comprises: a base layer; and an adhesive layer formed on a surface of the base layer, the adhesive layer has a wave pattern such that 95% or less of the area of the surface of the base layer is covered by the adhesive of the adhesive layer, wherein, the patch has an elongation of at least 120% in both machine direction and cross-machine direction.

## Description

### Field of the Invention

The present invention provides a patch that has an excellent elongation in both machine direction and cross-machine direction. In particular, the present invention provides a composite metal far-infrared medical patch that has an excellent elongation in both machine direction and cross-machine direction

### Descriptions of the Related Art

In the field of sport medical treatment, the Kinesio tape is a kinesiology tape, which is an elastic patch that has good stretch ability and air permeability. The Kinesio tape can be applied to the human body to drag the soft tissues such as the fascia and the muscle of the human body to generate specific force and induce physiological effects, thereby providing the efficacy of supporting and protecting injured parts, relieving pain, and facilitating particular action or movement, etc.

However, due to the obstacle of a gluing technique regarding the back adhesive layer of patches, the existing patches are stretchable in only one direction, for example, only in machine direction or cross-machine direction, and thus have limitations in use.

### SUMMARY OF THE INVENTION

The present invention provides a patch, which has an excellent four-way elongation that the existing patches do not have, thereby allowing more flexibility in applications. The four-way elongation means that the patch can be stretched in both machine direction and cross-machine direction. In addition, the patch of the present invention can further comprise far-infrared fibers to emit far-infrared rays to increase the volume and rate of the user's blood flow, thereby promoting blood circulation and relieving feelings of discomfort. Also, the patch of the present invention can further comprise an anti-bacterial material to provide an anti-bacterial effect. Thus, the present invention involves the following inventive objectives.

An objective of the present invention is to provide a patch, which comprises:
a base layer; and an adhesive layer formed on a surface of the base layer, wherein the adhesive layer has a wave pattern such that 95% or less of the area of the surface of the base layer is covered by the adhesive layer, wherein, the patch has an elongation of at least 120% in both machine direction and cross-machine direction.

In some embodiments of the present invention, the patch has an elongation ranging from 120% to 300% in both machine direction and cross-machine direction.

In some embodiments of the present invention, 50% to 80% of the area of said surface of the base layer is covered by the adhesive layer.

In some embodiments of the present invention, the base layer comprises an anti-bacterial material on its surface, and the anti-bacterial material is selected from the group consisting of zinc oxide, titanium dioxide, silver oxide, and combinations thereof.

In some embodiments of the present invention, the base layer is a far-infrared base layer, for example, a fabric comprising far-infrared fibers.

In some embodiments of the present invention, the base layer is a fabric comprising far-infrared fibers, and the far-infrared fibers comprise the following elements: titanium (Ti), germanium (Ge), zinc (Zn), aluminum (Al), and magnesium (Mg), and the far-infrared fibers do not comprise the following elements: scandium (Sc), vanadium (V), chrome (Cr), cobalt (Co), and antimony (Sb).

In some embodiments of the present invention, the base layer is a fabric comprising far-infrared fibers, and the proportion of the far-infrared fibers is more than 0% and 50% or less based on the total number of the fibers of the far-infrared base layer.

In some embodiments of the present invention, the base layer is a fabric comprising elastic fibers in both warp direction and weft direction, the proportion of the elastic fibers in warp direction is more than 0% and 12% or less based on the total number of the warp fibers of the fabric, and the proportion of the elastic fibers in weft direction is more than 0% and 12% or less based on the total number of the weft fibers of the fabric.

In some embodiments of the present invention, the base layer is a fabric comprising elastic fibers in both warp direction and weft direction, and the elastic fibers are selected from one or more of polyester fibers and polyurethane fibers.

To render the above objectives, technical features and advantages of the present invention more apparent, the present invention will be described in detail with reference to some embodiments hereinafter.

### BRIEF DESCRIPTION OF THE DRAWINGS

Fig. 1 is a schematic view of the pattern of adhesive layer of the patch according to an embodiment of the present invention.
Fig. 2 is a schematic view of the gluing equipment particularly designed for preparing the patch of the present invention.
Fig. 3 is a chart showing the assessment results of pain felt by the subjects.
Fig. 4 is a chart showing the assessment results of distress caused by numbness in the hands and feet of the subjects.
Fig. 5 is a chart showing the assessment results of distress caused by several physiological conditions of the subjects.

### DESCRIPTION OF THE PREFERRED EMBODIMENTS

Hereinafter, some embodiments of the present invention will be described in detail. The present invention may be embodied in various embodiments and should not be limited to the embodiments described in the specification.

For clarity, the size of each element and each area in the appended drawings may be exaggerated and not depicted in actual proportion.

Unless it is additionally explained, the expressions "a," "the," or the like recited in the specification and the claims should include both the singular and the plural forms.

As used herein, the term "elongation" refers to a ratio of the length of the patch after stretching to the length of the patch before stretching and is shown in percentage (%). For example, if a patch has a length of 10 cm in machine direction before stretching and a length of 15 cm in machine direction after stretching, the elongation of the patch in machine direction is 150%.

As used herein, the term "elastic fiber" refers to a fiber with an elongation of at least 125%.

As compared to the prior art, the present invention features the patch of the present invention that has an adhesive layer with a wave pattern formed on a surface of the base layer of the patch by using a special gluing process, such that the patch of the present invention has an excellent elongation in both machine direction and cross-machine direction. In addition, the base layer of the patch of the present invention can be a far-infrared base layer, wherein the far-infrared base layer comprises far-infrared fibers making the patch of the present invention be capable of emitting far-infrared rays to promote blood circulation and relieving the affected part's discomfort. In some embodiments of the present invention, the patch of the present invention is a composite metal far-infrared patch for medical treatment. Hereinafter, the structure and preparing method of the patch of the present invention will be described in detail.

### 1. Patch

The patch of the present invention comprises a base layer and an adhesive layer formed on a surface of the base layer. The patch of the present invention has an elongation of at least 120% in both machine direction and cross-machine direction. The patch of the present invention preferably has an elongation ranging from 120% to 300% in both machine direction and cross-machine direction, for example, an elongation of 125%, 130%, 135%, 140%, 145%, 150%, 155%, 160%, 165%, 170%, 175%, 180%, 185%, 190%, 195%, 200%, 205%, 210%, 215%, 220%, 225%, 230%, 235%, 240%, 245%, 250%, 255%, 260%, 265%, 270%, 275%, 280%, 285%, 290%, or 295%, or within a range between any two of the values described herein. As described above, the elongation refers to a percentage value of the length of the patch after stretching to the length of the patch before stretching.

### 1.1. Base layer

In the patch of the present invention, the base layer comprises elastic fibers, which impart excellent elongation to the patch. In some embodiments of the present invention, the base layer is a far-infrared base layer which comprises far-infrared fibers. As used herein, the patch comprising a far-infrared base layer is also called a far-infrared patch.

The base layer of the patch of the present invention can be a fabric or a non-woven fabric, and can be prepared by several weaving methods (or machines) or forming methods of non-woven fabrics known in the art. The weaving methods include but are not limited to a knitting method and a braiding method. The forming methods of non-woven fabrics include but are not limited to a spun-bond method, a water lace method, a needle punch method, a chemical bond method and a thermal bond method. In some embodiments of the present invention, the base layer is a far-infrared base layer and is a fabric formed by blend-weaving far-infrared fibers, elastic fibers, and general fibers through a knitting method, wherein the general fibers refer to fibers that do not emit far-infrared rays and are not elastic.

### 1.1.1. Elastic fiber

As used herein, an elastic fiber means a synthetic fiber that has high fracture elongation and high elastic recovery. Specifically, elastic fibers are one or more selected from polyester fibers and polyurethane fibers. Said polyester fibers include but are not limited to thermoplastic polyester elastomer (TPEE). In the appended examples, Spandex (a highly elastic fiber invented by DuPont in United States) and TPEE are used.

The base layer of the patch of the present invention is a fabric that comprises elastic fibers in both warp direction and weft direction. As used herein, the elastic fibers in warp direction and weft direction are also called warp elastic fibers and weft elastic fibers, respectively. In some embodiments of the present invention, based on the total number of the warp fibers of the fabric, the proportion of the elastic fibers in warp direction is more than 0% and 12% or less, such as 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, or 11.5%, or within a range between any two of the values described herein. In some embodiments of the present invention, based on the total number of the weft fibers of the fabric, the proportion of the elastic fibers in weft direction is more than 0% and 12% or less, such as 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, or 11.5%, or within a range between any two of the values described herein. A patch comprising more than 12% of the elastic fibers in warp direction and weft direction is not preferred in terms of comfort and air permeability.

### 1.1.2. Far-infrared fiber

As used herein, far-infrared fibers are fibers that can emit far-infrared rays. Far-infrared fibers comprise a polymer matrix and far-infrared fillers dispersed in the polymer matrix. In some embodiments of the present invention, the far-infrared fillers contain the following elements: titanium (Ti), germanium (Ge), zinc (Zn), aluminum (Al), and magnesium (Mg), and the far-infrared fillers preferably do not contain the following elements: scandium (Sc), vanadium (V), chrome (Cr), cobalt (Co), and antimony (Sb). The far-infrared fillers with the above elementary composition can emit far-infrared rays with a wavelength perfectly suitable for humans, i.e., far-infrared rays with a wavelength ranging from 2 µm to 22 µm, particularly from 4 µm to 14 µm, and more particularly from 6 µm to 6.5 µm.

In addition to the aforementioned elements, the far-infrared fillers can further comprise other elements that can emit far-infrared rays; for example, the far-infrared fillers can further comprise one or more elements selected from the group consisting of silicon (Si), copper (Cu), calcium (Ca), iron (Fe), barium (Ba), potassium (K), sodium (Na), manganese (Mn), nickel (Ni) and gallium (Ga).

The polymer matrix of the far-infrared fibers is not particularly limited, and examples of the polymer matrix include one or more polymers selected from the group consisting of polyester (e.g., polyethylene terephthalate (PET), polybutylene terephthalate (PBT), and TPEE), polyurethane (PU), poly(vinyl chloride) (PVC), poly propylene (PP), polyamide (PA), amino group-containing polymers (e.g., polyethylenimine (PEI)), and silicone. In some embodiments of the present invention, the polymer matrix of the far-infrared fibers comprises TPEE.

The shape of the far-infrared fibers is not particularly limited. For example, the cross-section perpendicular to the long-axis of the far-infrared fibers can be circular, elliptic, triangular, quadrangular or other polygonal, X-shaped, Y-shaped, or cross-shaped, but the present invention is not limited thereto. Furthermore, far-infrared fibers that are hollow and thus lightweight and more elastic may be preferred.

The manufacturing method of the far-infrared fibers is not particularly limited. For example, the far-infrared fibers can be prepared by providing far-infrared fillers in the form of oxygen-containing compounds of the aforementioned far-infrared emitting elements, carbon-containing compounds of the aforementioned far-infrared emitting elements, oxygen and carbon-containing compounds of the aforementioned far-infrared emitting elements, or amino group-containing compounds of the aforementioned far-infrared emitting elements, combining a polymer matrix with the far-infrared fillers and manufacturing the far-infrared fibers by using conventional manufacturing methods for fiber synthesis, such as a full granulation method, masterbatch method, and injection method.

In the far-infrared patch of the present invention, based on the total number of the fibers of the far-infrared base layer, the proportion of the far-infrared fibers (i.e., fibers that emit far-infrared rays) is more than 0% and 50% or less, such as 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, or 49%, or within a range between any two of the values described herein, but the present invention is not limited thereto.

As described above, the polymer matrix of the far-infrared fibers of the far-infrared base layer of the present invention can be an elastic material, such as polyurethane (PU) and thermoplastic polyester elastomer (TPEE). Therefore, the far-infrared fibers can also serve as elastic fibers.

### 1.1.3. General fiber

In addition to the far-infrared fibers and elastic fibers, the patch of the present invention can further comprise general fibers that do not emit far-infrared rays and are not elastic. Examples of the general fibers include but are not limited to cotton fibers, polyester fibers, PU fibers, PVC fibers, PP fibers, PA fibers, and silicone fibers. In some embodiments of the present invention, the base layer is a far-infrared base layer, and based on the total number of the fibers of the far-infrared base layer, the proportion of general fibers is more than 0% and 52% or less, such as 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, or 51%, or within a range between any two of the values described herein, but the present invention is not limited thereto.

### 1.1.4. Anti-bacterial material

The base layer can optionally comprise an anti-bacterial material to provide anti-bacterial efficacy. As used herein, the anti-bacterial material refers to a material that can destroy microbes or inhibit microbe's growth. Examples of said anti-bacterial material include but are not limited to zinc oxide, titanium dioxide, and silver oxide. The aforementioned anti-bacterial materials can either be used alone or in a combination of two or more. In some embodiments of the present invention, the anti-bacterial material is attached onto the surface of the fibers of the base layer via post-processing. The anti-bacterial material preferably has a nanoparticle size, for example, the anti-bacterial material may have an average particle size of 10 nm to 100 µm. Examples of said post-processing include precipitation, spraying, sol-gel, thermal evaporation deposition, vacuum sputter deposition, and the like. The post-processing is preferably vacuum sputter deposition, and during the sputter deposition, there is preferably a blocking sheet covering the surface of the base layer that is away from the target material, such that more anti-bacterial materials can be attached to the surface of the base layer. Examples of said blocking sheet include but are not limited to plastic sheets, papers, and cloths.

### 1.2. Adhesive layer

The patch of the present invention comprises an adhesive layer formed on a surface of the base layer. The adhesive layer is formed by coating an adhesive material onto a surface of the base layer. Examples of the adhesive material include but are not limited to natural rubber, synthetic isoprene rubber, styrene-butadiene rubber (SBR), styrene-isoprene-styrene (SIS) block copolymer, styrene-butadiene-styrene (SBS) block copolymer, polyisobutylene, styrene-ethylene-butylene-styrene (SEBS) copolymer, styrene-ethylenepropylene-styrene (SEPS) copolymer, (meth)acrylate-based copolymer, silicone rubber, silicone resin, dimethyl siloxane, diphenyl siloxane, polyvinyl ethers, polyvinyl esters, ethylene-vinyl acetate (EVA) copolymer, and polyesters. In the appended examples, (meth)acrylate-based copolymer (e.g., acrylic adhesives) or silicone resin is used.

In the present invention, the adhesive material is coated onto a surface of the base layer by means of a special adhesive coating method, such that the formed patch can have an excellent elongation in both machine direction and cross-machine direction. By contrast, conventional patches are stretchable in only machine direction or cross-machine direction.

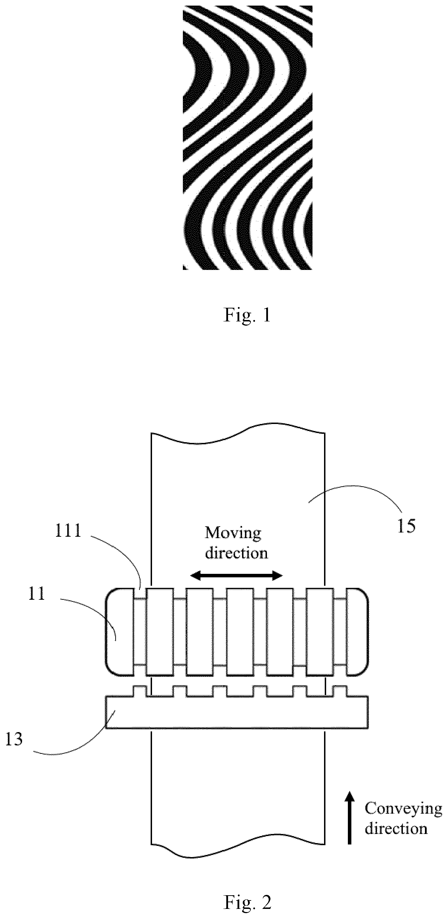

First, as known by persons having ordinary skill in the art, in conventional patches, a surface of the bottom layer of the patch is usually fully covered with the adhesive layer. That is, 100% of the area of said surface of the bottom layer of conventional patch is covered with the adhesive layer. As such, even though the conventional patches may contain elastic fibers, they have immensely limited elongation. By contrast, the adhesive layer of the patch of the present invention has a wave pattern, as shown in Fig. 1, and based on the whole area of the surface of the base layer to which the adhesive layer attached, the area covered by the adhesive layer is only 95% or less. In some embodiments of the present invention, based on the whole area of the surface of the base layer to which the adhesive layer attached, the area covered by the adhesive layer is 50% to 80%, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, or 79%, or within a range between any two of the values described herein.

In addition, in the patch of the present invention, the adhesive layer with a wave pattern is formed on the base layer by means of an improved comma coating process. Fig. 2 is a schematic view of the gluing equipment particularly designed for preparing the patch of the present invention. The gluing equipment comprises a coating roller 11 and a blade roller 13. The coating roller 11 is used to coat an adhesive material onto a surface of the base layer 15 and has grooves 111, and the blade roller 13 is used to scrape away the excess adhesive material in the grooves 111. The difference between conventional comma coating process and the improved comma coating process according to the present invention lies in that, the coating roller 11 and the blade roller 13 synchronously move in a periodic reciprocating motion about the center point of the base layer 15 along a moving direction perpendicular to the conveying direction of the base layer 15. The width of the grooves 111 is 1 (one) mm to 6 mm, such as 1.5 mm, 2 mm, 2.5 mm, 3 mm, 3.5 mm, 4 mm, 4.5 mm, 5 mm, or 5.5 mm. The moving frequency of the coating roller 11 and blade roller 13 is 0.5 mm/min to 0.6 mm/min. The coating roller 11 and blade roller 13 move in such a way that the maximum shift of the centers of the coating roller 11 and blade roller 13 with respect to the center of the periodic reciprocating motion is 4 mm to 8 mm. The conveying rate of the base layer 15 is 120 m/hr to 135 m/hr. By means of the improved comma coating process, the patch of the present invention can have an excellent elongation not only in machine direction but also in cross-machine direction. In some embodiments of the present invention, in order to avoid material waste, the width of the coating roller 11 is preferably greater than the width of the base layer, for example, 3 cm greater than the width of the base layer on each side.

### 2. Example

### 2.1. Preparation of far-infrared patch

Far-infrared fibers, elastic fibers and general fibers were blend-woven to a fabric through a knitting method. The fabric was used as a far-infrared base layer, wherein the proportion of far-infrared fibers is 12% based on the total number of the fibers of the fabric, the proportion of the elastic fibers in warp direction is 4% based on the total number of the warp fibers of the fabric, and the proportion of the elastic fibers in weft direction is 4% based on the total number of the weft fibers of the fabric.

An adhesive layer with a wave pattern was formed onto the far-infrared base layer using the improved comma coating process to prepare a far-infrared patch. The component of the adhesive layer is acrylic adhesives. The conditions of the comma coating process are as follows: the width of the grooves on the coating roller is 2.5 mm, the moving frequency of the coating roller and blade roller is 0.54 mm/min, the coating roller and blade roller move in such a way that the maximum shift of the centers of the coating roller and blade roller with respect to the center of the periodic reciprocating motion is 6 mm, and the conveying rate of the far-infrared base layer is 128 m/hr.

### 2.2. Elasticity test

### 2.2.1. Elongation of patches cut along longitudinal direction

As used herein, cutting a patch along the longitudinal direction means that the far-infrared patch of the present invention is cut in parallel with the machine direction of the far-infrared patch, such that the length direction of the far-infrared patch corresponds to the machine direction. The far-infrared patch of the present invention cut in longitudinal direction has the shape of a rectangle with a length of 10 cm and a width of 3 cm.

First, the elongation in length direction of the far-infrared patch cut in a longitudinal direction was measured, wherein a far-infrared patch cut in a longitudinal direction was fixed at one side along the length direction, and a force of 600 g or 1000 g was applied on the other side of the far-infrared patch along the length direction. The length of said far-infrared patch after stretching the patch with a force of 600 g is 18 cm, and the length of said far-infrared patch after stretching the patch with a force of 1000 g is 19.4 cm. Thus, the elongation in length direction of the far-infrared patch cut in longitudinal direction after stretching the patch with a force of 600 g is 180%, and the elongation in length direction of the far-infrared patch cut in a longitudinal direction after stretching the patch with a force of 1000 g is 194%.

Afterwards, the elongation in width direction of the far-infrared patch cut in a longitudinal direction was measured, wherein a far-infrared patch cut in a longitudinal direction was fixed at one side along the width direction, and a force of 600 g or 1000 g was applied on the other side of the far-infrared patch along the width direction. The width of the far-infrared patch after stretching the patch with a force of 600 g is 5.6 cm, and the width of the far-infrared patch after stretching the patch with a force of 1000 g is 6 cm. Thus, the elongation in width direction of the far-infrared patch cut in a longitudinal direction after stretching the patch with a force of 600 g is 187%, and the elongation in width direction of the far-infrared patch cut in a longitudinal direction after stretching the patch with a force of 1000 g is 200%.

### 2.2.2. Elongation of patches cut along transverse direction

As used herein, cutting a patch along the transverse direction means that the far-infrared patch of the present invention is cut perpendicular to the machine direction of the far-infrared patch, such that the length direction of the far-infrared patch corresponds to the transverse direction (i.e., the cross-machine direction). The far-infrared patch of the present invention cut in transverse direction has the shape of a rectangle with a length of 10 cm and a width of 3 cm.

First, the elongation in length direction of the far-infrared patch cut in a transverse direction was measured, wherein a far-infrared patch cut in a transverse direction was fixed at one side along the length direction, and a force of 600 g or 1000 g was applied on the other side of the far-infrared patch along the length direction. The length of said far-infrared patch after stretching the patch with a force of 600 g is 15.2 cm, and the length of said far-infrared patch after stretching the patch with a force of 1000 g is 16.5 cm. Thus, the elongation in length direction of the far-infrared patch cut in transvers direction after stretching the patch with a force of 600 g is 152%, and the elongation in length direction of the far-infrared patch cut in a transverse direction after stretching the patch with a force of 1000 g is 165%.

Afterwards, the elongation in width direction of the far-infrared patch cut in a transverse direction was measured, wherein a far-infrared patch cut in a transverse direction was fixed at one side along the width direction, and a force of 600 g or 1000 g was applied on the other side of the far-infrared patch along the width direction. The width of the far-infrared patch after stretching the patch with a force of 600 g is 4.6 cm, and the width of the far-infrared patch after stretching the patch with a force of 1000 g is 5 cm. Thus, the elongation in width direction of the far-infrared patch cut in transverse direction after stretching the patch with a force of 600 g is 153%, and the elongation in width direction of the far-infrared patch cut in a transverse direction after stretching the patch with a force of 1000 g is 167%.

### 2.3. Efficacy test

### 2.3.1. Test method

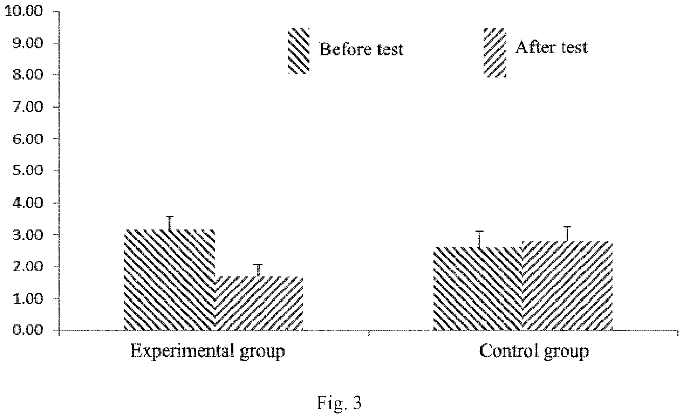

To evaluate the medical treatment effects of the far-infrared patch of the present invention, 66 subjects were randomly separated into an experimental group and a control group by the inventors of the present invention. Among the 66 subjects, 30 subjects were male and 36 subjects were female, and the age distribution of the 66 subjects is shown in the following Table 1. First, the 66 subjects were subjected to pain assessment and life quality assessment. Then, one roll of the far-infrared patch prepared from the aforementioned example was provided to each subject in the experimental group along with the usage instructions and safety precautions of a clinical trial nurse or coordinator. The subjects in the control group did not receive the far-infrared patch. After one week, the 66 subjects were subjected to pain assessment and life quality assessment again, and the assessment results were graphed as shown in Fig.3 to Fig. 5.

**Table 1: Age distribution of subjects**

| Age | Experimental group | Control group | Sum |
|---|---|---|---|
| 26 to 30 years old | 1 | 1 | 2 |
| 31 to 35 years old | 1 | 1 | 2 |
| 46 to 50 years old | 2 | 1 | 3 |
| 51 to 55 years old | 3 | 4 | 7 |
| 56 to 60 years old | 8 | 7 | 15 |
| 61 to 65 years old | 4 | 4 | 8 |
| 66 to 70 years old | 4 | 2 | 6 |
| 71 to 75 years old | 7 | 7 | 14 |
| 76 to 80 years old | 4 | 5 | 9 |
| Sum | 34 | 32 | 66 |

### 2.3.2. Data analysis

Fig. 3 is a chart showing the assessment results of pain felt by the subjects, wherein numerals 0 to 10 were used to assess the pain level of the subject, the larger the numeral value the higher the pain level, 0 represents feeling no pain, and 10 represents feeling the most serious pain. As can be seen from Fig. 3, the pain level of the subjects in the experimental group were significantly lowered after the subjects used the far-infrared patch of the present invention for one week. By contrast, the pain level of the subjects in the control group was slightly increased.

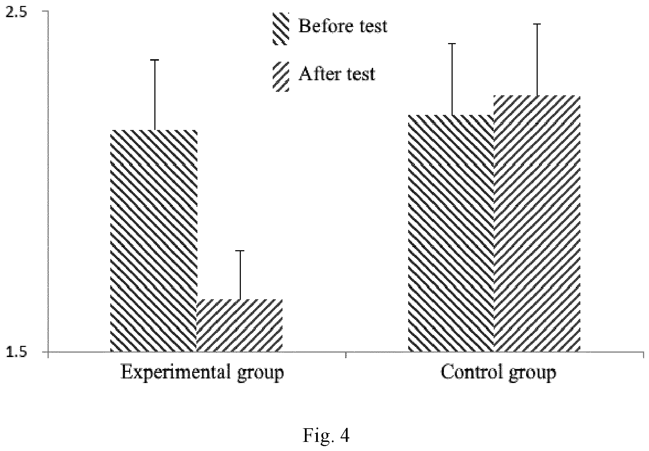

Fig. 4 is a chart showing the assessment results of distress caused by numbness in hands and feet of the subjects, wherein numerals 1 (one) to 5 were used to assess the level of distress caused by numbness in hands and feet of the subjects, the larger the numeral value the higher the level of distress, 1 (one) represents feeling no distress, and 5 represents feeling the most serious distress. As can be seen from Fig. 4, the distress level caused by numbness in hands and feet of the subjects in the experimental group were significantly lowered after the subjects used the far-infrared patch of the present invention for one week. By contrast, the distress level caused by numbness in hands and feet of the subjects in the control group was slightly increased.

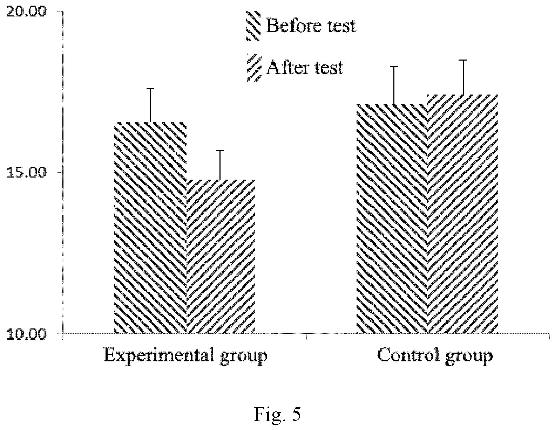

Fig. 5 is a chart showing the assessment results of distress caused by several physiological conditions of the subjects, wherein the following 8 physiological conditions were assessed: (1) muscle soreness, (2) chest pain, (3) cramp, (4) itchy skin, (5) skin dryness, (6) numbness in hands and feet, (7) nausea or an upset stomach, and (8) venous fistula, and wherein numerals 1 (one) to 5 were used to assess the level of distress caused by the aforementioned physiological conditions of the subjects, the larger the numeral value the higher the distress level, 1 (one) represents feeling no distress, 5 represents feeling the most serious distress, and the sum of the 8 physiological conditions is 40 at most. As can be seen from Fig. 5, the level of distress caused by the aforementioned physiological conditions of the subjects in the experimental group were significantly lowered after the subjects used the far-infrared patch of the present invention for one week. By contrast, the level of distress caused by the aforementioned physiological conditions of the subjects in the control group was slightly increased.

As can be seen from Fig. 3 to Fig. 5, the pain level and the level of distress caused by physiological conditions of the subjects in the experimental group were significantly decreased after the subjects used the far-infrared patch of the present invention for one week.

The above examples are used to illustrate the principle and efficacy of the present invention and show the inventive features thereof but are not used to limit the scope of the present invention. Therefore, the scope of protection of the present invention is that as defined in the claims as appended.

### BRIEF DESCRIPTION OF REFERENCE NUMERALS

11: coating roller
13: blade roller
15: base layer
111: groove

## Claims

1. A patch, which comprises:
a base layer; and an adhesive layer formed on a surface of the base layer, wherein
the adhesive layer has a wave pattern such that 95% or less of the area of said surface of the base layer is covered by the adhesive layer, wherein, the patch has an elongation of at least 120% in both machine direction and cross-machine direction.

2. The patch of claim 1, wherein the patch has an elongation ranging from 120% to 300% in both machine direction and cross-machine direction.

3. The patch of claim 1 or 2, wherein 50% to 80% of the area of said surface of the base layer is covered by the adhesive layer.

4. The patch of any one of claims 1 to 3, wherein the base layer comprises an anti-bacterial material on its surface.

5. The patch of claim 4, wherein the anti-bacterial material is selected from the group consisting of zinc oxide, titanium dioxide, silver oxide, and combinations thereof.

6. The patch of any one of claims 1 to 5, wherein the base layer is a far-infrared base layer.

7. The patch of claim 6, wherein the far-infrared base layer is a fabric comprising far-infrared fibers.

8. The patch of claim 7, wherein the far-infrared fibers comprise the following elements: titanium (Ti), germanium (Ge), zinc (Zn), aluminum (Al), and magnesium (Mg), and the far-infrared fibers do not comprise the following elements: scandium (Sc), vanadium (V), chrome (Cr), cobalt (Co), and antimony (Sb).

9. The patch of claim 7, wherein the proportion of the far-infrared fibers is more than 0% and 50% or less based on the total number of the fibers of the far-infrared base layer.

10. The patch of any one of claims 1 to 9, wherein the base layer is a fabric comprising elastic fibers in both warp direction and weft direction, the proportion of the elastic fibers in warp direction is more than 0% and 12% or less based on the total number of the warp fibers of the fabric, and the proportion of the elastic fibers in weft direction is more than 0% and 12% or less based on the total number of the weft fibers of the fabric.

11. The patch of claim 10, wherein the elastic fibers are selected from one or more of polyester fibers and polyurethane fibers.
